# EUROPEAN PATENT SPECIFICATION

(11) **EP 3 370 434 B1**
(45) Date of publication and mention of the grant of the patent: **11.03.2020**
(21) Application number: 16890905.9
(22) Date of filing: 20.07.2016
(51) Int. Cl.: H04R 1/10

(54) **EARPHONE AND INTERACTIVE SYSTEM**
KOPFHÖRER UND INTERAKTIONSSYSTEM
ÉCOUTEUR ET SYSTÈME INTERACTIF

(43) Date of publication of application: 05.09.2018
(73) Proprietor: Shenzhen Goodix Technology Co., Ltd., Futian Free Trade Zone Shenzhen, Guangdong 518000 (CN)
(72) Inventor: LIU, Chang, Shenzhen Guangdong 518000 (CN); YANG, Wangwang, Shenzhen Guangdong 518000 (CN); DUAN, Hongliang, Shenzhen Guangdong 518000 (CN); DENG, Gengchun, Shenzhen Guangdong 518000 (CN); WANG, Haixiang, Shenzhen Guangdong 518000 (CN)
(74) Representative: Kraus & Weisert Patentanwälte PartGmbB
(86) International application number: PCT/CN2016/090604
(87) International publication number: WO 2018/014246

(56) References cited:
- EP-A2- 2 728 899
- WO-A1-2016/046550
- CN-A- 104 080 028
- CN-A- 105 120 404
- CN-A- 105 451 115
- CN-A- 105 451 115
- CN-A- 105 491 483
- CN-A- 105 554 611
- US-A1- 2016 094 906
- US-A1- 2016 154 622
- Robert Triggs: "3.5mm audio vs USB Type-C: the good, bad and the future", https://www.androidauthority.com/3-5mm-aud io-usb-type-c-701507/, 12 July 2016 (2016-07-12), XP055513105, Retrieved from the Internet: URL:https://www.androidauthority.com/3-5mm -audio-usb-type-c-701507/ [retrieved on 2018-10-08]

## Description

### TECHNICAL FIELD

Embodiments of the present invention relate to the field of wearable devices, and in particular, relate to a headphone and an interaction system.

### BACKGROUND

Headphones are an entertainment tool which is frequently used by people, and are small in size and convenient to wear. Therefore, the headphones are widely used in people's life and work. For example, people may listen to music via the headphones while they are doing morning exercise, and may wear the headphones to watch videos, enjoy music and practice their English listening when they are going to work or going home after work.

However, with the development and advancement of science and technology, the function of the headphone is not limited to the single function of a traditional headphone. Smart headphones are nowadays being used among people. For example, smart headphones capable of detecting heart rate information of human bodies by detecting vibration at the auricle are well populated.

The biological features may be categorized into physiological features (for example, fingerprint, face image, iris, palm print and the like) and behavior features (for example, gait, voice, handwriting and the like). The biological features detection signifies identification and identity authentication of an individual based on the unique biological features of the individual.

At present, during practice of biological features detection and identification using the headphone in the prior art, the headphone is connected to a smart terminal such as a mobile phone, and enables the biological features detection function upon receiving an instruction of the smart terminal. However, during practice of the present invention, the inventors have found that the interaction between the smart terminal and the headphone is mainly based on a 3.5 mm headphone interface in the prior art, and only the analog audio protocol may be implemented. Therefore, the extensibility is poor, and if biological features detection needs to be implemented using the headphone, the headphone may only be inserted into a dedicated headphone socket of the smart terminal such as the mobile phone and the like.

CN105451115A discloses a headphone. The connection member 10 of the headphone for connecting the headphone and a smart terminal has four ends: i.e., a ground end 11, a first end 12 for connecting the smart terminal to a mode switching module 31 of the headphone, a second end 13 and a third end 14 for connecting the smart terminal to the left sound channel and right sound channel of the headphone loudspeaker 22. The headphone 30 includes: a loudspeaker 22, a microphone 21, a biological feature detection module 33; the mode switching module 31 for switching a working mode of the headphone supporting or not supporting biological feature detection according to a instruction of a control module 34; and a first communication module 35 for carrying out communication between the headphone and a smart terminal. The control module 34 detects and determines whether an instruction is received from the smart terminal, and controls, according to the instruction received from the smart terminal, the biological feature detection module 33, the mode switching module 31, the power management module 32 and the first communication module 35 to work. Biological feature data detected by the biological feature detection module 33 are transmitted to the smart terminal via the first communication module 35. The power management module 32 is configured to supply power to modules in the headphone 30. The mode switching module 31 is configured to select the connection of the first end 12 with the microphone 21 or connection with the first communication module 35, to determine whether the headphone 30 is in the working mode supporting the biological feature detection mode. When the first end 12 is connected to the microphone 21 via the mode switching module 31, the headphone is in the call mode; and when the first end 12 is connected to the first communication module 35 via the mode switching module 31, the headphone is in the biological feature detection mode.

### SUMMARY

Embodiments of the present invention are intended to provide a headphone and an interaction system, to at least solve the above technical problem in the prior art.

To achieve the objective of embodiments of the present invention, embodiments of the present invention provide a headphone according to claim 1.

Embodiments of the present invention further provide an interaction system according to claim 11.

The present invention has the following technical advantages:
The headphone includes: a Type-C interface, a controller electrically connected to the Type-C interface, a processor connected to the controller, a microphone electrically connected to the processor, and a loudspeaker electrically connected to the processor. The processor includes a biological features detection module. The biological features detection module is configured to detect biological features of a user wearing the headphone; and the controller is configured to control communication between the Type-C interface and the processor to control detection of biological features and processing of audio and video data. Since the Type-C interface not only supports analog communication, but also supports digital communication and analog-digital hybrid communication, no dedicated headphone socket is needed during practice of biological features detection using the headphone; instead, the Type-C interface may be directly used, which optimizes extensibility of the headphone.

### BRIEF DESCRIPTION OF THE DRAWINGS

FIG. 1 is a schematic structural diagram of a headphone according to Embodiment 1 of the present invention;
FIG. 2 is a schematic structural diagram of a headphone according to Embodiment 2 of the present invention;
FIG. 3 is a schematic structural diagram of a headphone according to Embodiment 3 of the present invention;
FIG. 4 is a schematic structural diagram of a headphone according to Embodiment 4 of the present invention;
FIG. 5 is a schematic structural diagram of a headphone according to Embodiment 5 of the present invention;
FIG. 6 is a schematic structural diagram of a headphone according to Embodiment 6 of the present invention;
FIG. 7 is a schematic structural diagram of a headphone according to Embodiment 7 of the present invention;
FIG. 8 is a schematic structural diagram of a headphone according to Embodiment 8 of the present invention;
FIG. 9 is a schematic structural diagram of a biological features detection module according to Embodiment 9 of the present invention;
FIG. 10 is a schematic structural diagram of a sensor module according to Embodiment 10 of the present invention;
FIG. 11 is a schematic structural diagram of an interaction system according to Embodiment 11 of the present invention;
FIG. 12 is a schematic structural diagram of a headphone according to Embodiment 12 of the present invention;
FIG. 13 is a schematic structural diagram of a headphone according to Embodiment 13 of the present invention; and
FIG. 14 is a schematic structural diagram of a headphone according to Embodiment 14 of the present invention.

### DETAILED DESCRIPTION

Practice of the present application is described in detail with reference to drawings and specific embodiments, such that the practice of addressing the technical problem using the technical means according to the present application and achieving the technical effects may be better understood and conducted.

In the embodiments of the present invention hereinafter, a headphone includes: a Type-C interface, a controller electrically connected to the Type-C interface, a biological features detection module connected to the controller, a microphone electrically connected to the controller, and a loudspeaker electrically connected to the controller. The biological features detection module is configured to detect biological features of a user wearing the headphone; and the controller is configured to control paring between the Type-C interface and a terminal and communication between the terminal and the biological features detection module, the microphone and the loudspeaker when the headphone is in a digital mode, to control detection of biological features and processing of audio and video data. Since the Type-C interface not only supports analog communication, but also supports digital communication and analog-digital hybrid communication, no dedicated headphone socket, is needed during detecting biological features with the headphone; instead, the Type-C interface may be directly used, which optimizes extensibility of the headphone.

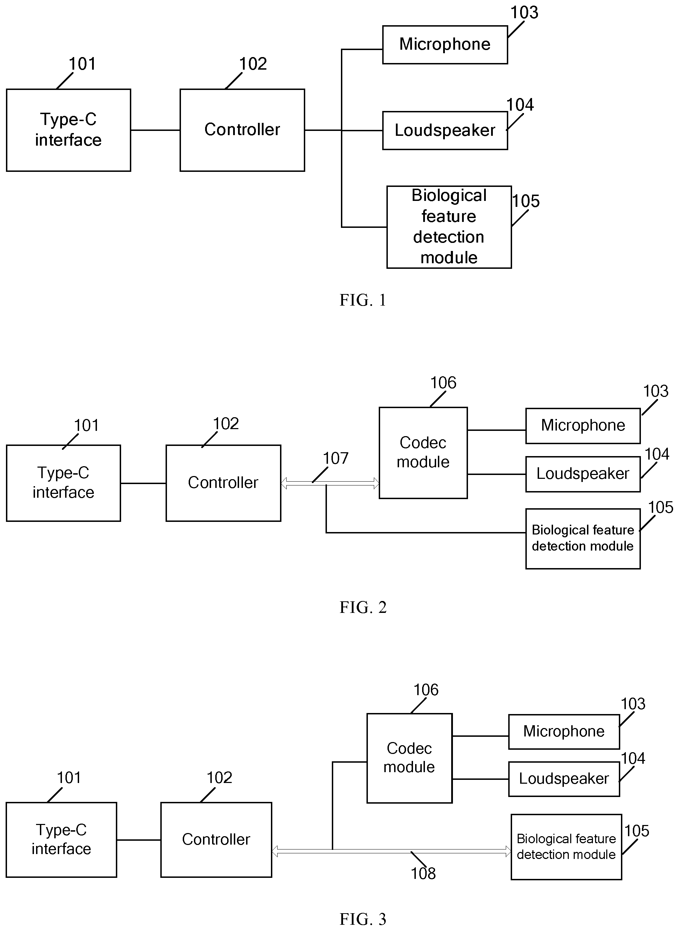

FIG. 1 is a schematic structural diagram of a headphone according to Embodiment 1 of the present invention. As illustrated in FIG. 1, the headphone includes: a Type-C interface 101, a controller 102 electrically connected to the Type-C interface 101, a biological features detection module 105 connected to the controller 102, a microphone 103 electrically connected to the controller, and a loudspeaker 104 electrically connected to the controller. The biological features detection module 105 is configured to detect biological features of a user wearing the headphone; and the controller 102 is configured to control paring between the Type-C interface 101 and a terminal and communication between the biological features detection module 105, the microphone 103 and the loudspeaker 104 when the headphone is in a digital mode, to control detection of biological features and processing of audio and video data to match the microphone 103 and the left and right amplifiers.

In this embodiment, the Type-C interface and the terminal are paired, such that the headphone and the terminal may identify each other and data may be transmitted there between.

In this embodiment, a plurality of biological features may be detected, for example, heart rate, step counting, body temperature, blood oxygen and the like. The duration of detecting biological features may involve monitoring biological features and parsing the monitored biological features.

In this embodiment, the microphone 103 may be configured to acquire sounds which may be audio and video analog signals; the loudspeaker 104 is configured to play the sounds, for example, when the headphone is connected to a smart terminal, the loudspeaker could be configured to play music or voice interactions for instant communication or the like, and convert digital signals corresponding to the music to audio and video analog signals to play, or convert the acquired audio and video analog signals by means of analog-to-digital conversion and digital-to-analog conversion to audio and video analog signals to play, which is not described herein any further.

In this embodiment, when the headphone is connected to a smart terminal, for example, in a wireless manner or in a wired manner, according to a control instruction of the smart terminal, the biological features detection module 105 is triggered to perform detection of biological features. The control instruction may be a voice control instruction, a mechanical key control instruction or the like, which is not described herein any further.

FIG. 2 is a schematic structural diagram of a headphone according to Embodiment 2 of the present invention. As illustrated in FIG. 2, different from Embodiment 1, in this embodiment, the headphone further includes a codec module 106. The codec module 106 is configured to communicate with the controller 102 via a first digital channel 107, and is configured to process audio and video analog signals and process audio and video digital signals; and the controller 102 is configured to communicate with the biological features detection module 105 by the first digital channel 107, to control detection of biological features.

In this embodiment, using a headphone based on the audio and video digital communication protocol as an example, when audio and video digital signals need to be played by the headphone, a connection is established between the first digital channel 107 and the codec module 106, to control process of audio and video digital data to match the microphone 103 and the loudspeaker 104. For example, when the headphone is connected to the smart terminal, music or voice interactions for instant communication or the like are played, and digital signals corresponding to the music are converted into audio and video analog signals and are then amplified to play by the loudspeaker 104; or audio and video analog signals acquired using the microphone 103 are converted by means of analog-to-digital conversion and digital-to-analog conversion to audio and video analog signals and are then amplified to play using the loudspeaker 104, which is not described herein any further.

In this embodiment, when the headphone is connected to a smart terminal, for example, in a wireless manner or in a wired manner, according to a control instruction of the smart terminal, when the biological features detection module 105 is triggered to perform detection of biological features, the controller 102 is connected to the biological features detection module 105 by the first digital channel 107, to control detection of biological features.

In this embodiment, the first digital channel 107 may be multiplexed by using a multiplexing switch, which is not described herein any further. The microphone 103 is connected to the codec module 106 via a wire of the microphone 103, and the loudspeaker 104 is connected to the codec module 106 via left and right sound channel wires, which is not described herein any further.

In this embodiment, the codec module may directly be configured to communicate with the controller via the first digital channel, so as to process the audio and video analog signals and process the audio and video digital signals. During practice of biological features detection, the controller is configured to communicate with the biological features detection module by the first digital channel.

FIG. 3 is a schematic structural diagram of a headphone according to Embodiment 3 of the present invention. As illustrated in FIG. 3, different from Embodiment 1, in this embodiment, the controller 102 is configured to communicate with the biological features detection module 105 via a second digital channel 108, to control detection of biological features and processing of audio and video data to match the microphone 103 and the loudspeaker 104. The headphone further includes a codec module 106. The codec module 106 is configured to communicate with the controller 102 by the second digital channel 108, and is configured to process audio and video analog signals and process audio and video digital signals.

In this embodiment, using a headphone based on audio and video digital communication protocol as an example, when the headphone is connected to a smart terminal, for example, in a wireless manner or in a wired manner, according to a control instruction of the smart terminal, if the biological features detection module 105 is triggered to perform detection of biological features, the controller 102 is connected to the biological features detection module 105 by the second digital channel 108, to control detection of biological features. When audio and video digital signals need to be played using the headphone, the second digital channel 108 is multiplexed to establish a connection with the codec module 106, to control processing of audio and video digital data to match the microphone 103 and the loudspeaker 104. For example, when the headphone is connected to the smart terminal, music or voice interactions for instant communication or the like are played, and digital signals corresponding to the music are converted into audio and video analog signals and are then amplified to play using the loudspeaker 104; or audio and video analog signals acquired using the microphone 103 are converted by means of analog-to-digital conversion and digital-to-analog conversion to audio and video analog signals and are then amplified to play using the loudspeaker 104, which is not described herein any further.

In this embodiment, the second digital channel 108 may be multiplexed by using a multiplexing switch, which is not described herein any further.

In this embodiment, during practice of biological features detection, the controller directly is configured to communicate with the biological features detection module via the first digital channel; and during practice of processing audio and video analog signals and processing of audio and video digital signals, the codec module may be configured to communicate with the controller via the first digital channel.

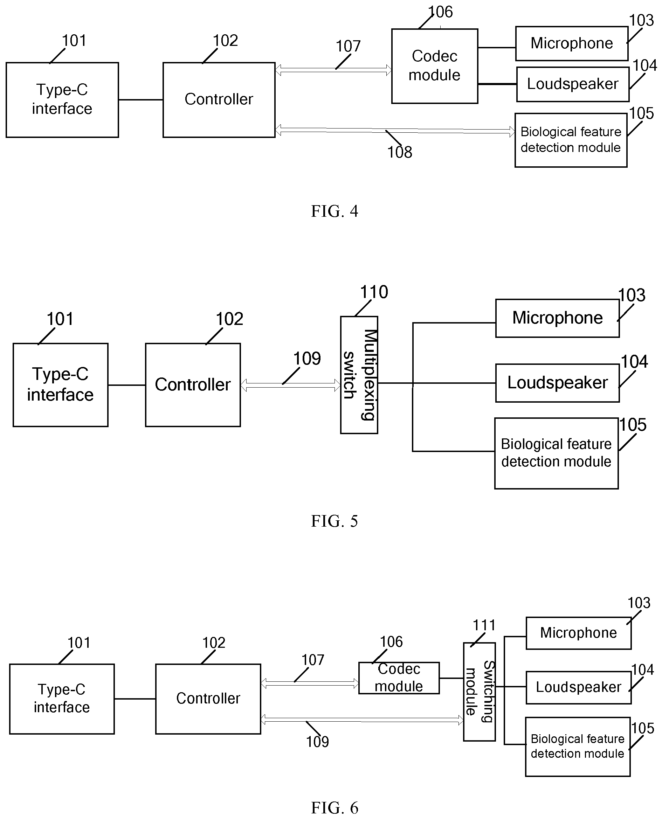

FIG. 4 is a schematic structural diagram of a headphone according to Embodiment 4 of the present invention. As illustrated in FIG. 4, different from Embodiment 1, in this embodiment, the processor further includes a codec module 106. The codec module 106 is configured to communicate with the controller 102 via a first digital channel 107, and is configured to process audio and video analog signals and process audio and video digital signals; and the controller 102 is configured to communicate with the biological features detection module 105 via a second digital channel 108, to control detection of biological features and processing of audio and video data to match the microphone 103 and the loudspeaker 104.

In this embodiment, using a headphone based on the audio and video digital communication protocol as an example, when audio and video digital signals need to be played using the headphone, a connection is established between the first digital channel 107 and the codec module 106, to control process of audio and video digital data to match the microphone 103 and the loudspeaker 104. For example, when the headphone is connected to the smart terminal, music or voice interactions for instant communication or the like are played, and digital signals corresponding to the music are converted into audio and video analog signals and are then amplified to play by the loudspeaker 104; or audio and video analog signals acquired using the microphone 103 are converted by means of analog-to-digital conversion and digital-to-analog conversion to audio and video analog signals and are then amplified to play using the loudspeaker 104, which is not described herein any further.

In this embodiment, when the headphone is connected to a smart terminal, for example, in a wireless manner or in a wired manner, according to a control instruction of the smart terminal, when the biological features detection module 105 is triggered to perform detection of biological features, the controller 102 is connected to the biological features detection module 105 via the second digital channel 108, to control detection of biological features.

In another embodiment, based on the embodiments as illustrated in FIG. 1 to FIG. 4, optionally, the Type-C interface 101 includes a first pin. The first pin is electrically connected to the controller 102, and is configured to supply power to the controller 102, the microphone 103 and the loudspeaker 104.

In another embodiment, based on the embodiments as illustrated in FIG. 1 to FIG. 4, optionally, the Type-C interface 101 includes a second pin. The second pin is electrically connected to the controller 102, and is configured to carry out communication between paring the headphone and the terminal using the headphone.

In another embodiment, based on the embodiments as illustrated in FIG. 1 to FIG. 4, optionally, the Type-C interface 101 includes a third pin. The third pin is electrically connected to the biological features detection features 105, and is configured to supply power to the biological features detection module 105.

In another embodiment, based on the embodiments as illustrated in FIG. 1 to FIG. 4, optionally, the Type-C interface 101 includes a plurality of fourth pins. The fourth pin is electrically connected to the controller 102, and is configured to carry out communication between the Type-C interface 101 and the biological features detection module 105, the microphone 103 and the loudspeaker 104.

FIG. 5 is a schematic structural diagram of a headphone according to Embodiment 5 of the present invention. As illustrated in FIG. 5, different from Embodiment 1, in this embodiment, the headphone further includes a first analog channel 109, and the controller 102 is electrically connected to the biological features detection module 105, the microphone 103 and the loudspeaker 104 via the first analog channel 109, to control detection of biological features and processing of audio and video data to match the microphone 103 and the loudspeaker 104.

Optionally, in this embodiment or any other embodiment, the headphone further includes a multiplexing switch 110. The multiplexing switch 110 is configured to control the biological features detection module 105, the microphone 103 and the loudspeaker 104 via multiplex the same first analog channel 109.

Optionally, in any embodiment of the present invention, a signal wire corresponding to the microphone 103 is multiplexed to supply power to the biological features detection module 105, and carry out uplink communication of the biological features detection module 105; and left and right sound channel wires corresponding to the loudspeaker 104 are multiplexed to carry out downlink communication of the biological features detection module 105. The uplink communication includes uploading detected biological features data and the like to the terminal such as a mobile phone and the like, and the downlink communication includes sending a control instruction and the like to the biological features detection module 105 by the terminal such as a mobile phone via the controller 102.

When audio and video analog signals need to be played using the headphone, the first analog channel 109 is connected to the microphone 103 and the loudspeaker 104. For example, when the headphone is connected to the smart terminal, music or voice interactions for instant communication or the like are played, and digital signals corresponding to the music are converted into audio and video analog signals and are then amplified to play using the loudspeaker 104; or audio and video analog signals acquired using the microphone 103 are converted by means of analog-to-digital conversion and digital-to-analog conversion to audio and video analog signals and are then amplified to play using the loudspeaker 104, which is not described herein any further.

In this embodiment, when the headphone is connected to a smart terminal, for example, in a wireless manner or in a wired manner, according to a control instruction of the smart terminal, when the biological features detection module 105 is triggered to perform detection of biological features, the controller 102 is connected to the biological features detection module 105 via the first analog channel 109, to control detection of biological features.

In another embodiment, based on the embodiment as illustrated in FIG. 5, optionally, the Type-C interface 101 includes a fourth pin and a fifth pin. The fourth pin and the fifth pin are respectively pulled down to the ground via a first pull-down resistor and a second pull-down resistor, such that the headphone is in an analog mode.

In another embodiment, based on the embodiment as illustrated in FIG. 5, optionally, the Type-C interface 101 includes a plurality of sixth pins. The sixth pin is connected to the first analog channel 109 via the controller 102, and is configured to carry out communication between the Type-C interface 101 and the biological features detection module 105, the microphone 103 and the loudspeaker 104.

FIG. 6 is a schematic structural diagram of a headphone according to Embodiment 6 of the present invention. As illustrated in FIG. 6, in this embodiment, the headphone further includes a first analog channel 109, and the controller 102 is electrically connected to the biological features detection module 105, the microphone 103 and the loudspeaker 104 via the first analog channel 109, to control detection of biological features and processing of audio and video data to match the microphone 103 and the loudspeaker 104.

The headphone further includes a codec module 106. The codec module 106 is configured to communicate with the controller 102 via a first digital channel 107, and is configured to process audio and video analog signals and process audio and video digital signals; and the controller 102 is configured to communicate with the biological features detection module 105 by the first digital channel 107, to control detection of biological features.

The headphone further includes a switching module. The switching module is configured to switch to detect of biological features and process audio and video data by the first analog channel 109 or the first digital channel 107, so as to match the microphone 103 and the loudspeaker 104.

In this embodiment, switching of the first analog channel and the first digital channel is controlled by using the switching module, thereby implementing transmission of both digital audio protocol and analog audio. In the aspect of audios, such devices as mobile phones and the like that are equipped with a built-in Hi-Fi module or supports analog audio output may employ analog audio transmission, and such devices as mobile phone and the like that support the digital audio protocol may employ digital audio transmission.

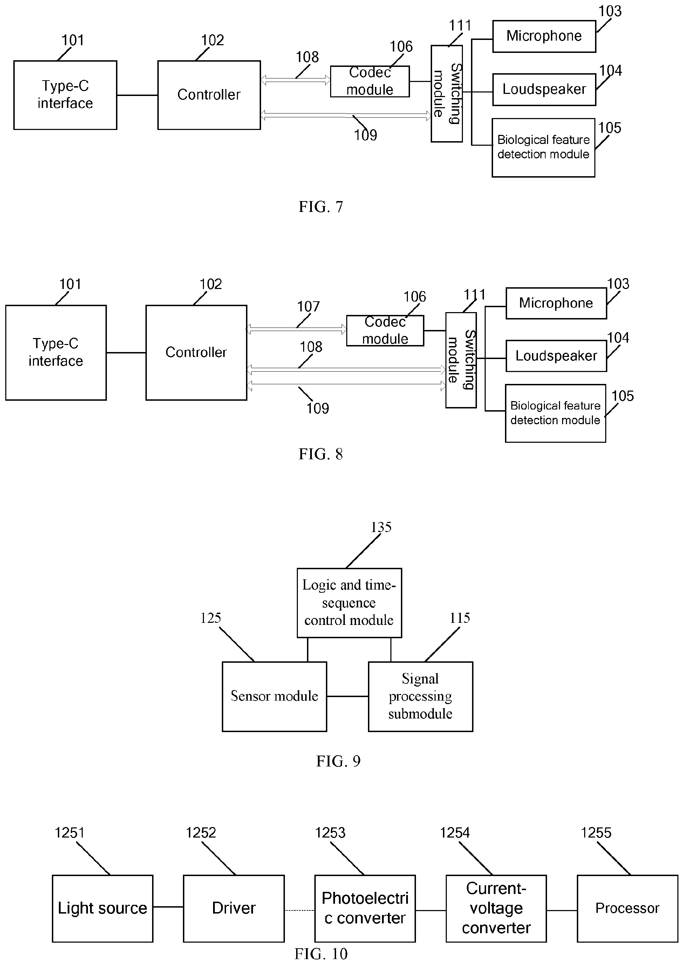

FIG. 7 is a schematic structural diagram of a headphone according to Embodiment 7 of the present invention. As illustrated in FIG. 7, using a hybrid configuration of the audio and video digital communication protocol and the audio and video analog communication protocol as an example, the headphone further includes a first analog channel 109. The controller 102 is electrically connected to the biological features detection module 105, the microphone 103 and the loudspeaker 104 via the first analog channel 109, to control detection of biological features and processing of audio and video data to match the microphone 103 and the loudspeaker 104. The controller 102 is configured to communicate with the biological features detection module 105 via a second digital channel 108, to control detection of biological features and processing of audio and video data to match the microphone 103 and the loudspeaker 104.

The headphone further includes a codec module 106. The codec module 106 is configured to communicate with the controller 102 by the second digital channel 108 and is configured to process audio and video analog signals and process audio and video digital signals.

The headphone further includes a switching module. The switching module is configured to switch to detect biological features and process audio and video data by the first analog channel 109 or the second digital channel 108, so as to match the microphone 103 and the loudspeaker 104.

In this embodiment, switching of the first analog channel and the second digital channel is controlled by using the switching module, thereby implementing transmission of both digital audio protocol and analog audio. In the aspect of audios, such devices as mobile phones and the like that are equipped with a built-in Hi-Fi module or supports analog audio output may employ analog audio transmission, and such devices as mobile phone and the like that support the digital audio protocol may employ digital audio transmission.

FIG. 8 is a schematic structural diagram of a headphone according to Embodiment 8 of the present invention. As illustrated in FIG. 8, using a hybrid configuration of the audio and video digital communication protocol and the audio and video analog communication protocol as an example, the processor further includes a codec module 106. The codec module 106 is configured to communicate with the controller 102 via a first digital channel 107, and is configured to process audio and video analog signals and process audio and video digital signals. The controller 102 is configured to communicate with the biological features detection module 105 via a second digital channel 108, to control detection of biological features and processing of audio and video data to match the microphone 103 and the loudspeaker 104.

The headphone further includes a first analog channel 109. The controller 102 is electrically connected to the biological features detection module 105, the microphone 103 and the loudspeaker 104 via the first analog channel 109, to control detection of biological features and processing of audio and video data to match the microphone 103 and the loudspeaker 104.

The headphone further includes a switching module. The switching module is configured to switch to detect biological features and process audio and video data by the first analog channel 109 or the first/second digital channel, so as to match the microphone 103 and the loudspeaker 104.

In this embodiment, switching of the first analog channel and the first/second digital channel is controlled by using the switching module, thereby implementing transmission of both digital audio protocol and analog audio. In the aspect of audios, such devices as mobile phones and the like that are equipped with a built-in Hi-Fi module or supports analog audio output may employ analog audio transmission, and such devices as mobile phone and the like that support the digital audio protocol may employ digital audio transmission.

FIG. 9 is a schematic structural diagram of a biological features detection module according to Embodiment 9 of the present invention. As illustrated in FIG. 9, the biological features detection module includes a signal processing submodule 115 and a sensor module 125. The sensor module is configured to detect the biological features; and the signal processing submodule 115 is configured to acquire the detected biological features and process the acquired biological features.

Optionally, in this embodiment, further including a logic and time-sequence control module 135, configured to perform time-sequence control on the sensor module and the signal processing submodule 115.

FIG. 10 is a schematic structural diagram of a sensor module according to Embodiment 10 of the present invention. As illustrated in FIG. 10, the sensor module includes: a light source 1251 configured to irradiate a detected region, a driver 1252 configured to drive the light source to emit light, an photoelectric converter 1253 configured to receive an optical signal reflected by the detected region and convert the optical signal into a current signal, a current-voltage converter 1254 configured to convert the current signal into a voltage signal, and a processor 1255 configured to process the voltage signal.

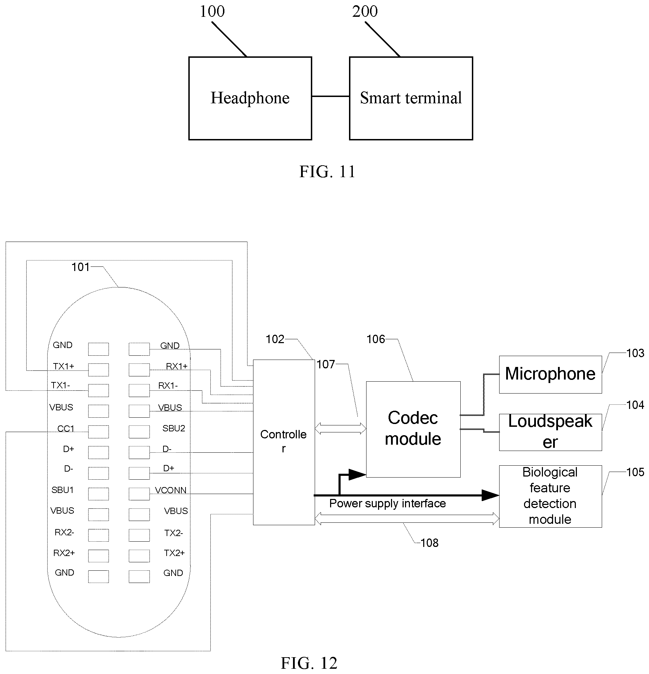

FIG. 11 is a schematic structural diagram of an interaction system according to Embodiment 11 of the present invention. As illustrated in FIG, 10, the interaction system includes a smart terminal 200 and a headphone 100 as described in any of the above embodiments. Processing of audio and video data and detection of biological features by the biological features detection module in the headphone are triggered by connecting the Type-C interface in the headphone to the smart terminal 200.

FIG. 12 is a schematic structural diagram of a headphone according to Embodiment 12 of the present invention. As illustrated in FIG. 12, corresponding to the specific implementation manner as illustrated in FIG. 4, in this embodiment, the processor further includes a codec module 106. The codec module 106 is configured to communicate with the controller 102 via a first digital channel 107, and is configured to process audio and video analog signals and process audio and video digital signals; and the controller 102 is configured to communicate with the biological features detection module 105 via a second digital channel 108, to control detection of biological features and processing of audio and video data to match the microphone 103 and the loudspeaker 104 to match the microphone 103 and the loudspeaker 104.

In this embodiment, the Type-C interface 101 is a Type-C male connector, and may specifically includes totally 24 pins including a Type-C male connector 101 (an connector between a headphone and a terminal device) supporting the USB interface, a Vconn pin, a D1+ pin, a D1- pin, a D2+ pin, a D2- pin, four VBUS pins, four GND pins, a CC1 pin, a CC2 pin (multiplexing the Vconn pin), a SBU1 pin, a SBU2 pin, an RX1+ pin, an RX1- pin, an RX2+ pin, an RX2- pin, a TX1+ pin, a TX1- pin, a TX2+ pin, a TX2- pin that are specified in the protocol of the Type-C interface 101. The D1+ pin, the D1- pin, the D2+ pin and the D2- pin are two pairs of D+s and D-s in the drawing.

In this embodiment, the CC2 pin of the Type-C interface 101 is configured to the Vconn pin and electrically connected to the controller 102, and is configured to supply power to the controller 102, the biological features detection module 105, the microphone 103 and the loudspeaker 104.

In this embodiment, the CC1 pin of the Type-C interface 101 is electrically connected to the controller 102, and is configured to carry out communication for paring the headphone and the terminal using the headphone.

In this embodiment, the VBUS pin of the Type-C interface 101 is electrically connected to the processor and the codec module 106 via the controller 102, and is configured to supply power to the biological features detection module 105 and the codec module 106.

In this embodiment, the TX1+, TX1-, RX1+, RX1-, D+ and D- pins of the Type-C interface 101 are electrically connected to the controller 102, and are configured to carry out communication between the Type-C interface 101 and the biological features detection module 105, the microphone 103 and the loudspeaker 104.

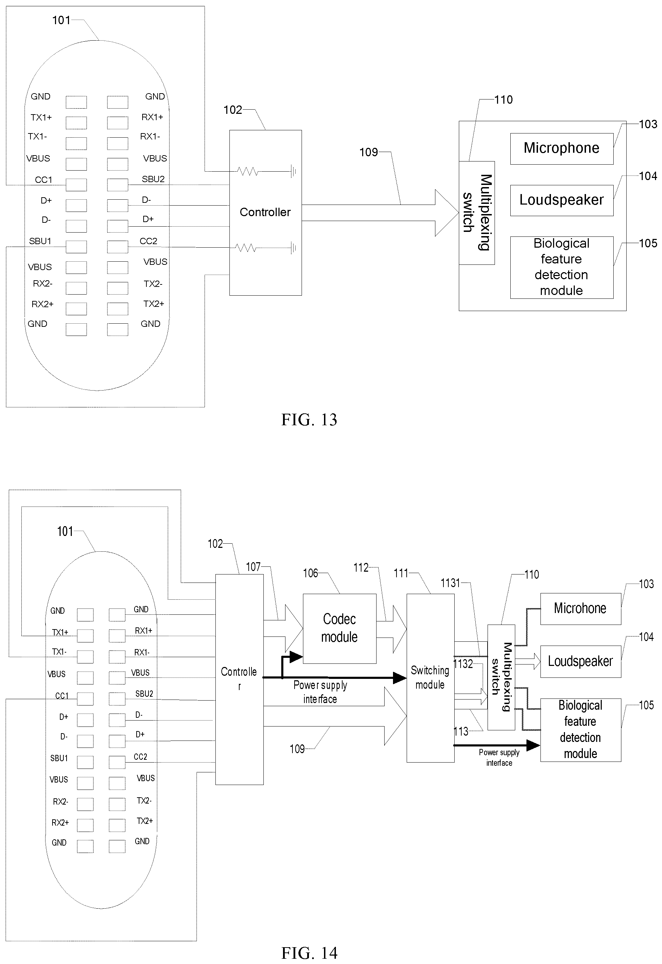

FIG. 13 is a schematic structural diagram of a headphone according to Embodiment 13 of the present invention. As illustrated in FIG. 13, in this embodiment, the headphone further includes a first analog channel 109, and the controller 102 is electrically connected to the biological features detection module 105, the microphone 103 and the loudspeaker 104 via the first analog channel 109, to control detection of biological features and processing of audio and video data to match the microphone 103 and the loudspeaker 104.

In this embodiment, the multiplexing switch 110 is configured to control the biological features detection module 105, the microphone 103 and the loudspeaker 104 to multiplex the same first analog channel 109.

In this embodiment, a signal wire corresponding to the microphone 103 is multiplexed to supply power to the biological features detection module 105, and carry out uplink communication of the biological features detection module 105; and left and right sound channel wires corresponding to the loudspeaker 104 are multiplexed to carry out downlink communication of the biological features detection module 105. Specifically, the first analog channel 109 may include a wire of the microphone 103, two sound channel wires, and a ground wire; the loudspeaker 104 is electrically connected to the two sound channel wires; and the microphone 103 is electrically connected to the wire of the microphone 103.

Like Embodiment 12 as described above, in this embodiment, the Type-C interface is a Type-C male connector, and may specifically includes totally 24 pins including a Type-C male connector 101 (an connector between a headphone and a terminal device) supporting the USB interface, a Vconn pin, a D1+ pin, a D1- pin, a D2+ pin, a D2- pin, four VBUS pins, four GND pins, a CC1 pin, a CC2 pin, a SBU1 pin, a SBU2 pin, an RX1+ pin, an RX1- pin, an RX2+ pin, an RX2- pin, a TX1+ pin, a TX1- pin, a TX2+ pin, a TX2- pin that are specified in the protocol of the Type-C interface 101. The D1+ pin, the D1- pin, the D2+ pin and the D2- pin are two pairs of D+s and D-s in the drawing.

In this embodiment, the CC1 pin and the CC2 pin of the Type-C interface 101 are pulled down to the ground via a first pull-down resistor and a second pull-down resistor, such that the headphone is in an analog mode.

In this embodiment, the GND, CC1, CC2, SBU1, SBU2, D+ and D- pins of the Type-C interface 101 are electrically connected to the first analog channel 109 via the controller 102, and are configured to carry out communication between the Type-C interface 101 and the biological features detection module 105, the microphone 103 and the loudspeaker 104.

FIG. 14 is a schematic structural diagram of a headphone according to Embodiment 14 of the present invention. As illustrated in FIG. 14, in this embodiment, the headphone further includes a codec module 106. The codec module 106 is configured to communicate with the controller 102 via a first digital channel 107, and is configured to process audio and video analog signals and process audio and video digital signals.

The headphone further includes a first analog channel 109. The controller 102 is electrically connected to the biological features detection module 105, the microphone 103 and the loudspeaker 104 via the first analog channel 109, to control detection of biological features and processing of audio and video data to match the microphone 103 and the loudspeaker 104.

The headphone further includes a switching module 111. The switching module 111 is configured to switch to detect biological features and process audio and video data by the analog channel or the first digital channel, so as to match the microphone 103 and the loudspeaker 104. The codec module 106 is configured to communicate with the switching module 111 via a second analog channel 112.

In this embodiment, the multiplexing switch 110 is configured to control the biological features detection module 105, the microphone 103 and the loudspeaker to multiplex the same third analog channel 113. The switching module is connected to the multiplexing switch 110 via the third analog channel 113. The third analog channel 113 includes a microphone wire 1131 and two sound channel wires 1132, and the two sound channel wires 1132 correspond to the left and right sound channel.

In a digital headphone mode, the second analog channel 112 is connected to the microphone wire 1131 and the two sound channel wires 1132; and in an analog headphone mode, the first analog channel 109 is connected to the microphone wire 1131 and the two sound channel wires 1132.

When the audio and video digital communication protocol is used, in this embodiment, the CC2 pin of the Type-C interface 101 is configured to the Vconn pin and connected to the controller 102, and is configured to supply power to the controller 102. The CC1 pin of the Type-C interface 101 is electrically connected to the controller 102, and is configured to carry out communication for paring the headphone and the terminal using the headphone. The VBUS pin of the Type-C interface 101 is electrically connected to the biological features detection module 105 and the codec module 106 via the controller 102, and is configured to supply power to the biological features detection module 105 and the codec module 106. The TX1+, RX1+, D+ and D- pins of the Type-C interface 101 are electrically connected to the controller 102, and are configured to carry out communication between the Type-C interface 101 and the processor.

When the audio and video analog communication protocol is used, in this embodiment, the CC1 pin and the CC2 pin of the Type-C interface 101 are pulled down to the ground via a first pull-down resistor and a second pull-down resistor respectively. The SBU1, SBU2, D+ and D-pins of the Type-C interface 101 are electrically connected to a first analog channel 109 via the controller 102, and are configured to carry out communication between the Type-C interface 101 and the processor.

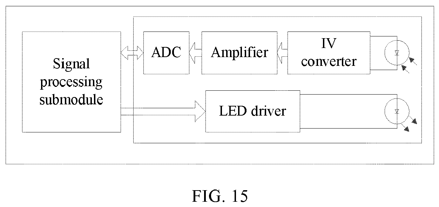

FIG. 15 is a schematic structural diagram of a biological features detection module according to Embodiment 15 of the present invention. As illustrated in FIG. 15, in this embodiment, using heart rate detection as an example, the biological features detection module 105 includes a signal processing submodule 115 and a sensor module 125. The sensor module 125 includes: an LED light source configured to irradiate a detected region of a user, an LED driver configured to drive the light source to emit light, an photoelectric converter configured to receive an optical signal reflected by the detected region and convert the optical signal into a current signal, an IV converter configured to convert the current signal into a voltage signal, and an ADC configured to process the voltage signal.

In this embodiment, an amplifier configured to amplify signals output by the IV converter may be added before the ADC.

The signal processing submodule 115 may judge a heart rate parameter of a tested object according to the regular variation of the strength of the reflected light, such that the biological features detection module 105 supports the heart rate detection function.

In conclusion, in the above embodiments of the present invention, since the Type-C interface not only supports analog communication, but also supports digital communication and analog-digital hybrid communication, no dedicated headphone socket is needed during practice of biological features detection using the headphone; instead, the Type-C interface may be directly used, which optimizes extensibility of the headphone. Biological features detection based on digital communication, analog communication and analog-digital hybrid communication may be implemented by pairing the Type-C interface and the smart terminal. In addition, since no dedicated circular headphone socket is needed ,the Type-C interface of the terminal such as the mobile phone may be multiplexed, one interface of the terminal is capable of supporting external headphones, charging and data transmission simultaneously and may be extended to such interfaces as an audio accessory/VGA/HDM/DP or the like. If an adapter is equipped, the Type-C interface may further support previous-generation interfaces such as USB3.0, USB2.0 and the like.

The apparatus according to the embodiments of the present application may be practiced by a computer program. A person skilled in the art should understand the above division of units and modules is only an exemplary one, and if the apparatus is divided into other units or modules or not divided, the technical solution shall also fall within the protection scope of the present application as long as the information object has the above functions.

A person skilled in the art shall understand that the embodiments of the present application may be described to illustrate methods, apparatuses (devices), or computer program products. Therefore, hardware embodiments, software embodiments, or hardware-plus-software embodiments may be used to illustrate the present application. In addition, the present application may further employ a computer program product which may be implemented by at least one non-transitory computer-readable storage medium with an executable program code stored thereon. The non-transitory computer-readable storage medium comprises but not limited to a disk memory, a CD-ROM, and an optical memory.

The present invention is described based on the flowcharts and/or block diagrams of the method, apparatus (device), and computer program product. It should be understood that each process and/or block in the flowcharts and/or block diagrams, and any combination of the processes and/or blocks in the flowcharts and/or block diagrams may be implemented using computer program instructions. These computer program instructions may be issued to a computer, a dedicated computer, an embedded processor, or processors of other programmable data processing device to generate a machine, which enables the computer or the processors of other programmable data processing devices to execute the instructions to implement an apparatus for implementing specific functions in at least one process in the flowcharts and/or at least one block in the block diagrams.

These computer program instructions may also be stored a non-transitory computer-readable memory capable of causing a computer or other programmable data processing devices to work in a specific mode, such that the instructions stored on the non-transitory computer-readable memory implement a product comprising an instruction apparatus, where the instruction apparatus implements specific functions in at least one process in the flowcharts and/or at least one block in the block diagrams.

These computer program instructions may also be stored on a computer or other programmable data processing devices, such that the computer or the other programmable data processing devices execute a series of operations or steps to implement processing of the computer. In this way, the instructions, when executed on the computer or the other programmable data processing devices, implement the specific functions in at least one process in the flowcharts and/or at least one block in the block diagrams.

Although the preferred embodiments of the present application are described above, once knowing the basic creative concept, a person skilled in the art can make other modifications and variations to these embodiments. Therefore, the appended claims are intended to be construed as covering the preferred embodiments and all the modifications and variations falling within the scope of the present application. Obviously, a person skilled in the art can make various modifications and variations to the present application without departing from the spirit and scope of the present application. In this way, the present application is intended to cover the modifications and variations if they fall within the scope of the appended claims.

## Claims

1. A headphone, comprising:
a USB-C interface (101);
a controller (102) electrically connected to the USB-C interface (101);
a biological features detection module (105) connected to the controller (102);
a microphone (103) electrically connected to the controller (102), and
a loudspeaker (104) electrically connected to the controller (102);
wherein the biological features detection module (105) is configured to detect biological features of a user wearing the headphone (100); the controller (102) is configured to control, when the headphone (100) is in a digital mode, paring between the USB-C interface (101) and a terminal (200) and communication among the terminal (200), the biological features detection module (105), the microphone (103) and the loudspeaker (104), to control detection of biological features and processing of audio data,
the headphone further comprising:
a first digital channel (107) and a codec module (106), wherein the codec module (106) is configured to communicate with the controller (102) via the first digital channel (107), and is configured to process audio analog signals and audio digital signals; , the controller (102) is configured to communicate with the biological features detection module (105) via the first digital channel (107) to control detection of biological features, or the controller (102) is configured to communicate with the biological features detection module (105) via a second digital channel (108) to control detection of biological features and processing of audio data;
an analog channel (109), wherein the controller (102) is electrically connected to the biological features detection module (105), the microphone (103) and the loudspeaker (104) via the analog channel (109) to control detection of biological features and processing of audio data; and
a switching module (111), wherein the switching module (111) is configured to switch to detect biological features and process audio data by the first digital channel (107) or the analog channel (109).

2. The headphone according to claim 1, wherein the controller (102) is configured to communicate with the biological features detection module (105) via the second digital channel (108) to control detection of the biological features and processing of audio data; the headphone (100) further comprises a codec module (106) that is configured to communicate with the controller (102) by the second digital channel (108) and process audio analog signals and process audio digital signals.

3. The headphone according to any one of claims 1 to 2wherein the USB-C interface (101) comprises a first pin, wherein the first pin is electrically connected to the controller (102) and is configured to supply power to the controller (102), the biological features detection module (105), the microphone (103) and the loudspeaker (104).

4. The headphone according to any one of claims 1 to 2, wherein the USB-C interface (101) comprises a second pin, wherein the second pin is electrically connected to the controller (102) and is configured to carry out communication if paring is performed between the headphone (100) and a terminal (200) using the headphone (100).

5. The headphone according to any one of claims 1 to 2, wherein the USB-C interface (101) comprises a third pin, wherein the third pin is electrically connected to the biological features detection module (105) and is configured to supply power to the biological features detection module (105) and the codec module (106).

6. The headphone according to any one of claims 1 to 2, wherein the USB-C interface (101) comprises a plurality of fourth pins, wherein the fourth pin is electrically connected to the controller (102) and is configured to carry out communication between the USB-C interface (101) and the biological features detection module (105), the microphone (103) and the loudspeaker (104).

7. The headphone according to claim 1, further comprising a multiplexing switch (110) configured to control the biological features detection module (105), the microphone (103) and the loudspeaker (104) to multiplex the same analog channel (109).

8. The headphone according to claim 7, wherein a signal wire corresponding to the microphone (103) is multiplexed to supply power to the biological features detection module (105), and carry out uplink communication of the biological features detection module (105); left and right sound channel wires corresponding to the loudspeaker (104) are multiplexed to carry out downlink communication of the biological features detection module (105).

9. The headphone according to claim 1, wherein the USB-C interface (101) comprises a fourth pin and a fifth pin, wherein the fourth pin and the fifth pin are respectively pulled down to the ground via a first pull-down resistor and a second pull-down resistor, such that the headphone (100) is in an analog mode.

10. The headphone according to claim 1, wherein the USB-C interface (101) comprises a plurality of sixth pins, wherein the sixth pin is connected to the analog channel (109) via the controller (102) and are configured to carry out communication between the USB-C interface (101) and the biological features detection module (105), the microphone (103) and the loudspeaker (104).

11. An interaction system, comprising a smart terminal (200) and the headphone (100) as defined in any one of claims 1 to 10; wherein processing of audio data and detection of biological features by the biological features detection module (105) in the headphone (100) are triggered by connecting the USB-C interface (101) in the headphone (100) to the smart terminal (200).

## Patentansprüche

1. Ein Kopfhörer, umfassend:
eine USB-C-Schnittstelle (101);
eine Steuereinheit (102), der elektrisch mit der USB-C-Schnittstelle (101) verbunden ist;
ein Modul zur Erkennung biologischer Merkmale (105), das an die Steuereinheit (102) angeschlossen ist;
ein Mikrofon (103), das elektrisch mit der Steuereinheit (102) verbunden ist, und
einen Lautsprecher (104), der elektrisch mit der Steuereinheit (102) verbunden ist;
wobei das Modul (105) zur Erfassung biologischer Merkmale konfiguriert ist, um biologische Merkmale eines den Kopfhörer (100) tragenden Benutzers zu erfassen; die Steuereinheit (102) konfiguriert ist, dass die Steuereinheit, wenn der Kopfhörer (100) in einem digitalen Modus ist, die Verbindung zwischen der USB-C-Schnittstelle (101) und einem Endgerät (200), und die Kommunikation zwischen dem Endgerät (200), dem Modul (105) zur Erfassung biologischer Merkmale, dem Mikrofon (103) und dem Lautsprecher (104) kontroliert, um die Erfassung biologischer Merkmale und die Verarbeitung von Audiodaten zu kontrollieren,
der Kopfhörer ferner umfassend:
einen ersten digitalen Kanal (107) und ein Codec-Modul (106), wobei das Codec-Modul (106) konfiguriert ist, um mit der Steuereinheit (102) über den ersten digitalen Kanal (107) zu kommunizieren, und um analoge Audiosignale und digitale Audiosignale zu verarbeiten; die Steuereinheit (102) konfiguriert ist, um mit dem Modul (105) zur Erkennung biologischer Merkmale über den ersten digitalen Kanal (107) zu kommunizieren, und weiter um die Erkennung biologischer Merkmale zu kontrollieren; oder die Steuereinheit (102) konfiguriert ist, um mit dem Modul (105) zur Erkennung biologischer Merkmale über einen zweiten digitalen Kanal (108) zu kommunizieren, und weiter um die Erkennung biologischer Merkmale und die Verarbeitung von Audiodaten zu kontrollieren;
einen Analogkanal (109), wobei die Steuereinheit (102) mit dem Modul (105) zur Erfassung biologischer Merkmale, dem Mikrofon (103) und dem Lautsprecher (104) über den Analogkanal (109) elektrisch verbunden ist, um die Erfassung biologischer Merkmale und die Verarbeitung von Audiodaten zu kontrollieren; und
ein Schaltmodul (111), wobei das Schaltmodul (111) zum Schalten konfiguriert ist, um die biologische Merkmale zu erfassen und die Audiodaten durch den ersten digitalen Kanal (107) oder den analogen Kanal (109) zu verarbeiten.

2. Kopfhörer nach Anspruch 1, wobei die Steuereinheit (102) konfiguriert ist, um mit dem Modul (105) zur Erfassung biologischer Merkmale über den zweiten digitalen Kanal (108) zu kommunizieren, und um die Erfassung der biologischen Merkmale und die Verarbeitung von Audiodaten zu kontrollieren; der Kopfhörer (100) umfasst ferner ein Codec-Modul (106), das konfiguriert ist, um mit der Steuereinheit (102) über den zweiten digitalen Kanal (108) zu kommunizieren, und die analogen Audiosignale und die digitalen Audiosignale zu verarbeiten.

3. Kopfhörer nach einem der Ansprüche 1 bis 2, wobei die USB-C-Schnittstelle (101) einen ersten Pin umfasst, wobei der erste Pin elektrisch mit der Steuereinheit (102) verbunden und konfiguriert ist, um die Steuereinheit (102), das Modul (105) zur Erfassung biologischer Merkmale, das Mikrofon (103) und den Lautsprecher (104) mit Strom zu versorgen.

4. Kopfhörer nach einem der Ansprüche 1 bis 2, wobei die USB-C-Schnittstelle (101) einen zweiten Pin umfasst, wobei der zweite Pin elektrisch mit der Steuereinheit (102) verbunden und konfiguriert ist, um eine Kommunikation auszuführen, wenn eine Verbindung zwischen dem Kopfhörer (100) und einem Anschluss (200) unter Verwendung des Kopfhörers (100) durchgeführt wird.

5. Kopfhörer nach einem der Ansprüche 1 bis 2, wobei die USB-C-Schnittstelle (101) einen dritten Pin umfasst, wobei der dritte Pin elektrisch mit dem Modul (105) zur Erkennung biologischer Merkmale verbunden und konfiguriert ist, um das Modul (105) zur Erkennung biologischer Merkmale und das Codec-Modul (106) mit Strom zu versorgen.

6. Kopfhörer nach einem der Ansprüche 1 bis 2, wobei die USB-C-Schnittstelle (101) mehrere vierte Pins umfasst, wobei der vierte Pin elektrisch mit der Steuereinheit (102) verbunden und konfiguriert ist, um die Kommunikation zwischen der USB-C-Schnittstelle (101) und dem Modul (105) zur Erfassung biologischer Merkmale, dem Mikrofon (103) und dem Lautsprecher (104) durchzuführen.

7. Kopfhörer nach Anspruch 1, ferner umfassend einen Multiplex-Schalter (110), der konfiguriert ist, um das Modul (105) zur Erfassung biologischer Merkmale, das Mikrofon (103) und den Lautsprecher (104) zu kontrollieren, und weiter um denselben analogen Kanal (109) zu multiplexen.

8. Kopfhörer nach Anspruch 7, wobei eine dem Mikrofon (103) entsprechende Signalleitung gemultiplext ist, um das Modul (105) zur Erfassung biologischer Merkmale mit Strom zu versorgen und eine Uplink-Kommunikation des Moduls (105) zur Erfassung biologischer Merkmale durchzuführen; linke und rechte Schallkanalleitungen, die dem Lautsprecher (104) entsprechen, gemultiplext sind, um eine Downlink-Kommunikation des Moduls (105) zur Erfassung biologischer Merkmale durchzuführen.

9. Kopfhörer nach Anspruch 1, wobei die USB-C-Schnittstelle (101) einen vierten Pin und einen fünften Pin aufweist, wobei der vierte Pin und der fünfte Pin jeweils über einen ersten Pull-Down-Widerstand und einen zweiten Pull-Down-Widerstand nach unten zur Masse gezogen werden, so dass der Kopfhörer (100) ist in einem analogen Modus.

10. Kopfhörer nach Anspruch 1, wobei die USB-C-Schnittstelle (101) mehrere sechste Pins aufweist, wobei der sechste Pin über die Steuereinheit (102) mit dem Analogkanal (109) verbunden und konfiguriert ist, um eine Kommunikation zwischen der USB-C-Schnittstelle (101) und dem Modul (105) zur Erfassung biologischer Merkmale, dem Mikrofon (103) und dem Lautsprecher (104) durchzuführen.

11. Interaktionssystem, umfassend ein intelligentes Terminal (200) und den Kopfhörer (100) wie in einem der Ansprüche 1 bis 10 definiert; wobei die Verarbeitung von Audiodaten und die Erfassung biologischer Merkmale durch das Modul (105) zur Erfassung biologischer Merkmale im Kopfhörer (100) durch Verbinden der USB-C-Schnittstelle (101) im Kopfhörer (100) mit dem intelligenten Terminal (200) ausgelöst werden.

## Revendications

1. Casque, comprenant:
une interface USB-C (101);
un contrôleur (102) relié électriquement à l'interface USB-C (101);
un module de détection de caractéristiques biologiques (105) relié au contrôleur (102);
un microphone (103) relié électriquement au contrôleur (102), et
un haut-parleur (104) relié électriquement au contrôleur (102);
dans lequel le module de détection de caractéristiques biologiques (105) est configuré pour détecter des caractéristiques biologiques d'un utilisateur portant le casque (100); le contrôleur (102) est configuré pour contrôler, lorsque le casque (100) est en mode numérique, l'association entre l'interface USB-C (101) et un terminal (200) et la communication entre le terminal (200), lemodule de détection de caractéristiques biologiques(105), le microphone (103) et le haut-parleur (104), pour contrôler la détection des caractéristiques biologiques et le traitement des données audio,
le casque comprenant en outre:
un premier canal numérique (107) et un module de codec (106), dans lequel le module de codec (106) est configuré pour communiquer avec le contrôleur (102) via le premier canal numérique (107), et est configuré pour traiter des signaux audio analogiques et signaux audio numériques; le contrôleur (102) est configuré pour communiquer avec le module de détection de caractéristiques biologiques (105) via le premier canal numérique (107) pour contrôler la détection de caractéristiques biologiques, ou le contrôleur (102) est configuré pour communiquer avec le module de détection de caractéristiques biologiques (105) via un deuxième canal numérique (108) pour contrôler la détection des caractéristiques biologiques et le traitement des données audio;
un canal analogique (109), dans lequel le contrôleur (102) est relié électriquement au module de détection de caractéristiques biologiques (105), au microphone (103) et au haut-parleur (104) via le canal analogique (109) pour contrôler la détection de caractéristiques biologiques et le traitement des données audio; et
un module de commutation (111), dans lequel le module de commutation (111) est configuré pour commuter pour détecter des caractéristiques biologiques et traiter des données audio par le premier canal numérique (107) ou le canal analogique (109).

2. Casque selon la revendication 1, dans lequel le contrôleur (102) est configuré pour communiquer avec le module de détection de caractéristiques biologiques (105) via le deuxième canal numérique (108) pour contrôler la détection des caractéristiques biologiques et le traitement des données audio; le casque (100) comprend en outre un module de codec (106) qui est configuré pour communiquer avec le contrôleur (102) par le deuxième canal numérique (108) et traiter les signaux audio analogiques et traiter les signaux audionumériques.

3. Casque selon l'une quelconque des revendications 1 à 2, dans lequel l'interface USB-C (101) comprend une première broche, dans lequel la première broche est reliée électriquement au contrôleur (102) et est configurée pour alimenter le contrôleur (102), le module de détection de caractéristiques biologiques (105), le microphone (103) et le haut-parleur (104).

4. Casque selon l'une quelconque des revendications 1 à 2, dans lequel l'interface USB-C (101) comprend une deuxième broche, dans lequel la deuxième broche est reliée électriquement au contrôleur (102) et est configurée pour effectuer une communication si l'association est effectuée entre le casque (100) et un terminal (200) utilisant le casque (100).

5. Casque selon l'une quelconque des revendications 1 à 2, dans lequel l'interface USB-C (101) comprend une troisième broche, dans lequel la troisième broche est reliée électriquement au module de détection de caractéristiques biologiques (105) et est configurée pour alimenter le module de détection de caractéristiques biologiques (105) et le module de codec (106).

6. Casque selon l'une quelconque des revendications 1 à 2, dans lequel l'interface USB-C (101) comprend une pluralité de quatrièmes broches, dans lequel la quatrième broche est reliée électriquement au contrôleur (102) et est configurée pour effectuer une communication entre l'interface USB-C (101) et le module de détection de caractéristiques biologiques (105), le microphone (103) et le haut-parleur (104).

7. Casque selon la revendication 1, comprenant en outre un commutateur de multiplexage (110) configuré pour contrôler le module de détection de caractéristiques biologiques (105), le microphone (103) et le haut-parleur (104) pour multiplexer le même canal analogique (109).

8. Casque selon la revendication 7, dans lequel un fil de signal correspondant au microphone (103) est multiplexé pour alimenter le module de détection de caractéristiques biologiques (105) et effectuer une communication en liaison montante du module de détection de caractéristiques biologiques (105); des fils des canaux sonores gauche et droit correspondant au haut-parleur (104) sont multiplexés pour effectuer une communication en liaison descendante du module de détection de caractéristiques biologiques (105).

9. Casque selon la revendication 1, dans lequel l'interface USB-C (101) comprend une quatrième broche et une cinquième broche, dans lequel la quatrième broche et la cinquième broche sont respectivement tirées vers le bas au sol via une première résistance de rappelet une seconde résistance de rappel, de telle sorte que le casque (100) est en mode analogique.

10. Casque selon la revendication 1, dans lequel l'interface USB-C (101) comprend une pluralité de sixièmes broches, dans lequel la sixième broche est reliée au canal analogique (109) via le contrôleur (102) et est configurée pour exécuter communication entre l'interface USB-C (101) et le module de détection de caractéristiques biologiques (105), le microphone (103) et le haut-parleur (104).

11. Système d'interaction, comprenant un terminal intelligent (200) et le casque (100) tel que défini dans l'une quelconque des revendications 1 à 10; dans lequel le traitement de données audio et la détection de caractéristiques biologiques par le module de détection de caractéristiques biologiques (105) dans le casque (100) sont déclenchés en connectant l'interface USB-C (101) dans le casque (100) au terminal intelligent (200).
